(19) **Europäisches Patentamt / European Patent Office / Office européen des brevets**

(11) **EP 4 696 769 A1**

(12) **EUROPEAN PATENT APPLICATION**
published in accordance with Art. 153(4) EPC

(43) Date of publication:
18.02.2026  Bulletin 2026/08

(21) Application number: 23937326.9

(22) Date of filing: 22.12.2023

(51) International Patent Classification (IPC):
$C12N\ 1/16^{(2026.01)}$    $A23K\ 10/12^{(2016.01)}$
$A23K\ 10/18^{(2016.01)}$    $A23K\ 10/30^{(2016.01)}$
$A23K\ 10/37^{(2016.01)}$    $A23L\ 27/24^{(2016.01)}$

(52) Cooperative Patent Classification (CPC):
A23K 10/12; A23K 10/18; A23K 10/30;
A23K 10/37; A23L 27/24; C12N 1/16;
C12R 2001/645; C12R 2001/74; C12R 2001/84;
C12R 2001/87

(86) International application number:
PCT/CN2023/140941

(87) International publication number:
WO 2024/234655 (21.11.2024 Gazette 2024/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority:  13.05.2023  CN 202310540113

(71) Applicant: Angel Yeast Co., Ltd
Yichang, Hubei 443003 (CN)

(72) Inventors:
• QIN, Xianwu
  Yichang, Hubei 443003 (CN)
• WU, Yexu
  Yichang, Hubei 443003 (CN)
• ZHANG, Yan
  Yichang, Hubei 443003 (CN)

• SUN, Yafang
  Yichang, Hubei 443003 (CN)
• LEI, Senlin
  Yichang, Hubei 443003 (CN)
• GUO, Tianfen
  Yichang, Hubei 443003 (CN)
• YU, Kui
  Yichang, Hubei 443003 (CN)
• HUANG, Xingfa
  Yichang, Hubei 443003 (CN)
• WANG, Chao
  Yichang, Hubei 443003 (CN)

(74) Representative: karo IP
Patentanwälte PartG mbB
Steinstraße 16-18
40212 Düsseldorf (DE)

Remarks:
The complete document including Reference
Table(s) and the Sequence Listing(s) can be
downloaded from the EPO website

(54) **METHOD FOR PRODUCING YEAST PRODUCT USING HIGH-LACTIC ACID INDUSTRIAL BY-PRODUCT**

(57)  Provided is a method for producing a yeast product using a high-lactic acid industrial by-product. The method comprises the following steps:(1) inoculating an activated yeast strain into a primary seed culture medium for fermentation culture to obtain a primary seed liquid; (2) inoculating the primary seed liquid into a secondary seed culture medium for fermentation culture to obtain a secondary seed liquid; (3) inoculating the secondary seed liquid into a fermentation culture medium for fermentation culture, wherein the fermentation culture medium comprises a high-lactic acid industrial by-product and/or a modified product of the high-lactic acid industrial by-product; and (4) separating the fermentation broth obtained in step (3) to obtain a yeast product, or drying the fermentation broth obtained in step (3) to obtain a yeast product. According to the method, the fermentation process is stable, the production efficiency is high, the product quality is stable, and the industrial application of using a high-lactic acid industrial by-product for producing a yeast is realized.

Fig. 1

## Description

## RELATED APPLICATION

The present application claims priority to the prior application document with application number 202310540113.X, filed in the China National Intellectual Property Administration on May 13, 2023, which is incorporated herein by reference in its entirety.

## Technical Field

The present invention relates to the technical field of yeast fermentation and in particular to a method for producing a yeast product using a high-lactic acid industrial by-product.

## Background Art

Corn steep liquor is a by-product of corn starch production and a water-soluble substance obtained during corn steeping. The corn steep liquor is rich in soluble proteins, growth factors, and some precursor substances, and contains about 40%-50% of dry matter. Corn steep water is a kind of high-concentration organic wastewater, which cannot directly be treated by the sewage treatment plant. Corn deep processing enterprises generally concentrate corn steep water into corn steep liquor and spray it onto corn fibers or corn germ meal to produce sprayed fibers or sprayed germ meal. The evaporation and drying process consumes a large amount of energy and has low added value. The excess corn steep liquor is used as an organic nitrogen source culture medium for the fermentation industry. In recent years, the processing capacity of corn deep processing enterprises has increased rapidly, and there is a serious oversupply of corn steep liquor. Enterprises need to subsidize shipping costs to process corn steep liquor, which seriously restricts the development of corn deep processing enterprises.

Distillers Dried Grains with Solubles (DDGS) is a kind of production waste liquid that remains after alcohol is distilled from corn fermented mash. It mainly contains protein, as well as fats, sugars, vitamins, etc. Distillers Dried Grains (DDG) liquor is rich in nutrients, but the treatment of DDGS waste liquid is difficult and costly.

Baijiu (Chinese liquor) yellow water is a small amount of downward moving water by gravity in the fermented grains, together with dissolved alcohols, aldehydes, acids, esters, minerals, reducing sugars, amino acids, etc., which is deposited at the bottom of the cellar and accumulated into a brownish liquid during Baijiu brewing. Direct disposal causes waste of resources and may also contaminate the environment.

Pickling water is a kind of industrial wastewater rich in lactic acid that is left after the industrial production of pickled vegetables, and the direct discharge will cause environmental pollution and is difficult to treat. However, in the process of pickle fermentation, a part of nutrients such as lactic acid, vitamins, calcium, phosphorus, and other minerals are released into the pickling water, and the direct discharge not only causes environmental pollution but also causes a part of the waste of nutrients.

The industrial by-products such as corn steep liquor, DDGS, Baijiu yellow water, or pickling water are rich in soluble protein, trace elements, lactic acid, and vitamins, with a lactic acid content of above 3%, which is a high-lactic acid industrial by-product. How to utilize it as a resource, turn waste into treasure, and reduce the pollution problem of enterprises has become a practical need for relevant enterprises.

Yeast products are fermented and propagated by yeast in a certain system and are widely used in the food, brewing, pharmaceutical, and feed industries. In this process, it is necessary to supply nutrients that are converted into cells and metabolites. Molasses is an essential raw material for yeast production, which not only provides the yeast with the necessary carbon source but also contains many trace elements and minerals that are necessary for yeast growth. In recent years, with the vigorous development of the yeast industry, the competition for molasses raw materials has become increasingly fierce. In order to ensure sufficient production of raw materials and reduce the cost of yeast production, it is one of the development directions of the yeast industry to find new cheap and efficient medium materials.

Chinese patent CN113455584A discloses a method for producing feed yeast granules using corn steep liquor, comprising strain activation, seed liquor preparation and fermenter production to obtain a fermentation product liquor, followed by multi-effect evaporation concentration and spray fluidized bed granulation to obtain a feed yeast granule product, wherein the yeast species is selected from one or two of *Saccharomyces cerevisiae, Candida utilis* or Baker's yeast, and the fermentation culture medium uses the corn steep liquor. This method produces feed yeast products with a crude protein content of ≥50% and a total of 18 amino acid content of ≥46%. Although it can meet the requirements of feed yeast, the product contains a large amount of unconverted corn steep liquor impurities, which can only be used as feed and cannot be used as a nitrogen source for microbial fermentation or the preparation of high-value yeast leachates.

## Summary of the Invention

The technical problem to be solved by the present invention is: the present invention provides a method for producing yeast using a high-lactic acid industrial by-product to produce a high protein yeast protein for use as a nitrogen source for antibiotic fermentation or to further produce yeast leachate to provide an organic nitrogen source for more microbial fermentation while achieving reasonable utilization of resources.

To achieve the above objectives, the present invention provides the following technical solution: The present application provides a method for producing a yeast product using a high-lactic acid industrial by-product comprising the following steps:

(1) inoculating an activated yeast strain into a primary seed culture medium for fermentation culture to obtain a primary seed liquid; wherein the yeast strain is selected from one or two or more of the group consisting of *Kluyveromyces marxianus, Kluyveromyces lactis, Pichia kudriavzeii* and *Candida tropicalis;*
(2) inoculating the primary seed liquid into a secondary seed culture medium for fermentation culture to obtain a secondary seed liquid;
(3) inoculating the secondary seed liquid into a fermentation culture medium for fermentation culture, wherein the fermentation culture medium comprises a high-lactic acid industrial by-product and/or a modified product of the high-lactic acid industrial by-product; and
(4) separating the fermentation broth obtained in step (3) to obtain a yeast product, or drying the fermentation broth obtained in step (3) to obtain a yeast product.

In some embodiments of the present application, in the method described above, the high-lactic acid industrial by-product of step (3) comprises one or two or more of the group consisting of corn steep liquor, Distillers Dried Grains with Solubles (DDGS), Baijiu yellow water, or pickling water, preferably corn steep liquor.

In some embodiments of the present application, the fermentation culture medium further comprises a nutrient, wherein the nutrient comprises a trace element and a vitamin.

In some embodiments of the present application, the trace element is selected from one or two or more of the group consisting of copper, calcium, iron, zinc, magnesium and potassium, preferably copper and/or calcium; preferably, the vitamin is selected from one or two or more of the group consisting of vitamin B1, vitamin B2, vitamin B3, vitamin B6, pantothenic acid, and vitamin B7, preferably one or two or more of the group consisting of vitamin B1, pantothenic acid, and vitamin B7.

In some embodiments of the present application, the nutrient comprises one or two or more of the group consisting of copper 2-5 ppm, calcium 400-1000 ppm, vitamin B1 10-30 ppm, pantothenic acid 15-30 ppm, and vitamin B7 0.5-1.5 ppm, based on the total weight of the fermentation culture medium.

In some embodiments of the present application, in the method described above, the modified product of the high-lactic acid industrial by-product is prepared by a process comprising the following steps: subjecting the high-lactic acid industrial by-product to enzymolysis with cellulase, enzymolysis with protease, and enzyme inactivation.

In some embodiments of the present application, in the method described above, the cellulase is added in an amount of 3-6 wt%o, preferably 4-6 wt%o, based on the dry weight of the high-lactic acid industrial by-product; preferably, the cellulase enzymolysis temperature is 55-65°C; preferably the enzymolysis time is 3-6 hours.

In some embodiments of the present application, in the method described above, the protease is added in an amount of 2-4 wt%o, preferably 2-3wt%o, based on the dry weight of the high-lactic acid industrial by-product; preferably, the temperature for the protease enzymolysis is 55-65°C; preferably the enzymolysis time is 3-6 hours.

In some embodiments of the present application, in the method described above, the enzyme inactivation comprises heating to 70-90°C and holding for 20-40 min.

In some embodiments of the present application, in the method described above, the fermentation culture of step (3) uses a stirred fermenter, with a stirring speed of 400-600 rpm, and preferably an aeration ratio of 1: 1-2; alternatively, the fermentation culture of step (3) uses an air-lift fermenter with an aeration ratio of 1: 10-70; preferably, the fermentation culture temperature is 25-35°C, and preferably, the culture time is 12-48 hours.

In some embodiments of the present application, in the method described above, the separation in step (4) comprises one or two or more of the group consisting of centrifugation, suction filtration, pressure filtration or plate-and-frame filtration, preferably centrifugation, more preferably the centrifugation speed is 3000-10000 rpm, preferably the centrifugation time is 5-20 min.

In some embodiments of the present application, the method further comprises the step of washing and/or drying the yeast product separated in step (4).

In some embodiments of the present application, in the method described above, the primary seed culture medium of step (1) is a YPD liquid medium; preferably, the fermentation culture temperature of step (1) is 25-35°C, preferably the fermentation culture time is 12-30 hours; more preferably, the speed is 150-220 rpm.

In some embodiments of the present application, in the method described above, the secondary seed culture medium of step (2) comprises glucose 5-8%, yeast leachate 0.3-0.6%, ammonium sulfate 1-2%, zinc sulfate 0.2-0.4%, potassium dihydrogen phosphate 0.4-0.6%, and magnesium sulfate 0.4-0.6% by mass volume percentage; preferably, the fermentation culture temperature of step (2) is 25-35°C; preferably the culture time is 12-36 hours; more preferably, the fermentation culture pH is 4.5-5.5; preferably the aeration is 2-4 L/min.

The present application also provides a yeast product obtainable by the above method, wherein the yeast product comprises 30-68% of protein by weight, preferably 50-68% of protein by weight; preferably, the yeast product comprises 1.5-9.0% of trehalose; more preferably, the yeast product contains 2.0-4.0% of phosphorus pentoxide.

In some embodiments of the present application, the yeast product is in the form of a powder, paste, or liquid.

The present application also provides use of the above-mentioned yeast product in a feed protein, food seasoning, or fermentation industrial nitrogen source.

Advantageous Effect of the Invention

The yeast product is produced by the method of the present invention, the fermentation process is stable, the production efficiency is high, and the product quality is stable. Compared with the prior art method for producing yeast using molasses, the method of the present invention is equivalent to the prior art in terms of production efficiency and quality, etc. but the cost is much lower than the prior art. Therefore, the industrial application of yeast for producing high-lactic acid industrial by-products can be realized by using the method of the present invention, increasing the channels for obtaining yeast sugar resources, widening the application range of high-lactic acid industrial by-products, and alleviating the problem of shortage of molasses resources in the yeast industry.

Strain deposit information

The strain *Kluyveromyces marxianus AMCC30634* (*Kluyveromyces marxianus AMCC30634*) used in the present application, was deposited at the China Collection of Type Cultures on February 27, 2023 with deposit number CCTCC NO: M 2023217, deposited at Wuhan University, Wuhan, China, postal code: 430072; Phone: 02768754052.

The strain *Kluyveromyces marxianus AMCC31342* (*Kluyveromyces marxianus AMCC31342*) obtain by mutagenesis in the present application, was deposited at the China Collection of Type Cultures on December 30, 2022 with deposit number CCTCC NO: M20222110, deposited at Wuhan University, Wuhan, China, postal code: 430072; Phone: (027)-68754052.

The *Saccharomyces cerevisiae* FX-2 (*Saccharomyces cerevisiae FX-2*) used in the present application was deposited at the Chinese Collection of Type Cultures (CCTCC) on August 1, 2016 with deposit number CCTCC NO: M2016418, deposited at: Wuhan University, Wuhan, China, postal code: 430072; Phone: (027)-68754052 (the *Saccharomyces cerevisiae* FX-2 has been described in the patent application of Chinese application number 201710532216.6 (publication number CN109207384A)).

The *Pichia kudriavzeii C4.12* (*Pichia kudriavzeii* C4.12) used in the present application was deposited at the Chinese Collection of Type Cultures (CCTCC) on January 21, 2021 with deposit number CCTCC NO: M 2021124, deposited at: Wuhan University, Wuhan, China, postal code: 430072; Phone: (027)-68754052 (the *Pichia kudriavzeii C4.12* strain has been described in the patent application of Chinese application number 202111509846.4 (publication number CN114107077A))

The *Kluyveromyces lactis AMCC 31347* (*Kluyveromyces lactis AMCC 31347*) used in the present application was deposited at the China Collection of Type Cultures (CCTCC) on February 27, 2023, with deposit number CCTCC NO: M2023218, deposited at: Wuhan University, Wuhan, China, postal code: 430072; Phone: (027)-68754052.

**Brief Description of the Drawings**

Fig. 1 is a schematic representation of the colony morphology of *Kluyveromyces marxianus AMCC30634;*

Fig. 2 is a schematic representation of the morphology of *Kluyveromyces marxianus AMCC30634* under a microscope;

Fig. 3 is a graph showing changes in wet weight of *Streptomyces avermitilis* cultured using yeast in Experimental Example 1 as a nitrogen source; and

Fig. 4 is a growth curve of *Escherichia coli* cultured using the yeast leachate of Experimental Example 2 as a nitrogen source.

## Detailed Description of the Invention

To make the purpose, technical solution, and technical effect of the embodiments of the present invention clearer, a clear and complete description of the technical solution in the embodiments of the present invention is provided. The described embodiments are a portion of the embodiments of the present invention and not all of the embodiments. Injunction with the embodiments of the present invention, all other embodiments obtained by a person of ordinary skill in the art without inventive effort fall within the scope of the present invention.

As used herein, mass volume percent refers to the mass content of a substance in a 100 mL solution, i.e. 1% = 1 g/100mL.

The term ppm as described herein is percent by weight.

The present application provides a *Kluyveromyces marxianus* (*Kluyveromyces marxianus*) with deposit number CCTCC NO: M 20222110. The *Kluyveromyces marxianus* has excellent resistance to organic acids and can be grown using lactic acid as a carbon source.

In the present application, the above-mentioned *Kluyveromyces marxianus* with deposit number CCTCC No: M 20222110 was obtained by mutagenesis using the strain with deposit number CCTCC NO: M 2023217 as the starting strain. The starting strain refers to the original strain used for the breeding treatment.

The strain CCTCC NO: M 2023217 in the application was isolated from fermented dairy products in the Xizang Autonomous Region. The strain has excellent organic acid resistance and can be grown using lactic acid as a carbon source. In addition, the strain has low pH tolerance.

In the present application, the ITS sequence of the strain with Deposit number CCTCC NO: M 2023217 is shown in SEQ ID NO: 3:

CCAACGGGGATTGCCTTAGTACGGCGAGTGAAGCGGCAAAAGCTCAAATTTGAAATC

TGGCGTCTTCGACGTCCGAGTTGTAATTTGAAGAAGGCGACTTTGTAGCTGGTCCTTG

TCTATGTTCCTTGGAACAGGACGTCATAGAGGGTGAGAATCCCGTGTGGCGAGGATC

CCAGTTATTTGTAAAGTGCTTTCGACGAGTCGAGTTGTTTGGGAATGCAGCTCTAAGT

GGGTGGTAAATTCCATCTAAAGCTAAATATTGGCGAGAGACCGATAGCGAACAAGTA

CAGTGATGGAAAGATGAAAAGAACTTTGAAAAGAGAGTGAAAAAGTACGTGAAATT

GTTGAAAGGGAAGGGCATTTGATCAGACATGGCGTTTGCTTCGGCTTTCGCTGGGCCA

GCATCAGTTTTAGCGGTTGGATAAATCCTCGGGAATGTGGCTCTGCTTCGGTAGAGTG

TTATAGCCCGTGGGAATACAGCCAGCTGGGACTGAGGATTGCGACTTTTGTCAAGGA

TGCTGGCGTAATGGTTAAATGCCGCCCGTCTTGAACCCACGGACCA

The present application also provides a *Kluyveromyces lactis* with deposit number CCTCC NO: M2023218. The *Kluyveromyces lactis* has excellent resistance to organic acids and can be grown using lactic acid as a carbon source.

The strain with the number CCTCC NO: M 2023218 described in the present application was isolated from fermented dairy product samples in the Inner Mongolia region.

In the present application, the ITS sequence of the strain with Deposit number CCTCC NO: M 2023218 is shown in SEQ ID NO: 4:

TCCGGGGAATCTACCTGATTTGAGGTCAACTTTGAGAGTTTTGATTAAGCCGTATGCC

TCAAGGAGACAAACACCAGCGAGTCTTTATAACACCTATAAGCTCATTGACCCTAGCT

TACCACGAATTGGCGCAAACCTAAGACGTAGATGTGCAAGAGTCGAGTCCATAGACT

TGACACGCAGCCCTGCTCACGCAGAAGGCAACGGCTAGCCACTTTCAAGTTAACCCG

AAAAACGAGTATCACTCACTACCAAACCCAAAGGTTTGAGAGAGAAATGACGCTCAA

ACAGGCATGCCCCCTGGAATACCAGAGGGCGCAATGTGCGTTCAAAGATTTGATGAT

TCACGAAAATCTGCAATTCACAATACATATCGCAATTCGCTGCGTTCTTCATCGATGC

GAGAACCAAGAGATCCGTTGTTGAAAGTTTTGAATATTAAATTTCTTGATAAATAGTT

TTTCATAATGCAAAATGTTGTTTGTGTTTATGTCCACTGGAGAGACGAGCTCTCCAGG

GAAGTAGTTCATAGAGAAAAAACTCCATTGTGTTTAGGATGAGAGAAAGAAAACTGA

TAGCAGAAAATCAAGAATTAGCCGCGCAATTAAGCGCAGGCCATATTCAGCGATTCC

CCCAGTAATCTACTCATTCATAATCTTTAATGATCCTTCCGCAGGTTCACCTACGGAA

ACCTTGTTACGATTTTTTACCTCCAA

In the present application, the ITS sequence refers to the Internal Transcribed Spacer sequence between the 5.8S rDNA and 28S rDNA in the rDNA gene. The length and sequence thereof vary greatly, and performing RFLP or sequence analysis on the amplification thereof can be used for classifying and identifying different biological types, strains, species, and genera of fungi. It is even used to distinguish very closely related species.

The present invention is primarily directed to the production of pure cultured yeast by fermentation of specific yeast species using a high-lactic acid industrial by-product as a substrate. On the one hand, in a specific embodiment of the present application, the present invention provides a method for producing yeast products using a high-lactic acid industrial by-product, comprising the following steps:

(1) inoculating an activated yeast strain into a primary seed culture medium for fermentation culture to obtain a primary seed liquid; wherein the yeast strain is selected from one or two or more of the group consisting of *Kluyveromyces marxianus, Kluyveromyces lactis, Pichia kudriavzeii,* and *Candida tropicalis;*

(2) inoculating the primary seed liquid into a secondary seed culture medium for fermentation culture to obtain a secondary seed liquid;

(3) inoculating the secondary seed liquid into a fermentation culture medium for fermentation culture, wherein the fermentation culture medium comprises a high-lactic acid industrial by-product and/or a modified product of the high-lactic acid industrial by-product; and

(4) separating the fermentation broth obtained in step (3) to obtain a yeast product, or drying the fermentation broth obtained in step (3) to obtain a yeast product.

Note that the heavy phase separated in step (4) of the present invention is the yeast product.

In some embodiments of the present application, the fermentation culture temperature of step (3) is 25-35°C, and preferably the culture time is 12-48 hours.

For example, the fermentation culture temperature may be 25°C, 28°C, 33°C, 35°C, etc.; the fermentation time may be 12 h, 24 h, 36 h, 48 h, etc., or a value within a range of values having any two of the above-mentioned values as endpoints.

Note that the fermenter for the fermentation culture of step (3) is not particularly limited and may be a stirred fermenter or an air-lift fermenter. When a stirred fermenter is used, the stirring speed is 400-600 rpm, and the aeration ratio is preferably 1: 1-2. When the rotation speed and aeration ratio are lower than this range, the dissolved oxygen of fermentation broth will be insufficient, the growth of yeast will be affected, and the final wet weight will be lower. When the rotation speed and aeration ratio are higher than these ranges, energy and compressed air are wasted, and bubble flooding is easy to occur in the fermentation process, which increases the risk of microbial contamination. When an air-lift fermenter is used, the aeration ratio is 1: 10-70.

The high-lactic acid industrial by-products referred to in the present invention refer to industrial by-products with a mass percentage of lactic acid of more than 3% and processed products thereof, including but not limited to by-product corn steep liquor for producing corn starch, by-product DDGS for producing alcohol, by-product Baijiu yellow water, or pickling water.

In some embodiments of the present application, the yeast strain is selected from one or two or more of the group consisting of *Kluyveromyces marxianus* with deposited number CCTCC NO: M *20222110, Kluyveromyces lactis* with deposited number CCTCC NO: M2023218, *Pichia kudriavzeii* C4.12 with deposited number CCTCC NO: M 2021124, and *Candida tropicalis* CGMCC 2.0588.

In some embodiments of the present application, in order to increase the content of sugars and amino acids in the fermentation broth, to be better absorbed and converted by the yeast so as to improve the fermentation efficiency and the quality of the yeast product, further comprising modifying the high-lactic acid industrial by-product to obtain a modified product of the high-lactic acid industrial by-product. The modified product of the high-lactic acid industrial by-product is obtainable by a method comprising the following steps:

a. dissolving the high-lactic acid industrial by-product in water according to 10-20% (w/v), adding cellulase 3-6‰ according to the dry weight of the high-lactic acid industrial by-product, and holding the temperature at 55-65°C for 3-6 hours;

b. then, adding protease 2-4‰ (based on the dry weight of the high-lactic acid industrial by-product), and holding the temperature at 55-65°C for 3-6 hours; and

c. heating to 70-90°C to inactivate the enzyme for 20-40 min.

For example, the amount of cellulase added may be 3‰, 4‰, 5‰, 6‰, etc. based on the dry weight of the high-lactic acid industrial by-product, or a value within a range of values having any two of the above-mentioned values as endpoints. The cellulase hydrolysis time may be 3 hours, 4 hours, 5 hours, 6 hours, etc., or a value within a range of values having any two of the above-mentioned values as endpoints.

For example, the protease may be added in an amount of 2‰, 2.5‰, 3‰, 3.5‰, 4‰, etc. based on the dry weight of the high-lactic acid industrial by-product, or a value within a range of values having any two of the above-mentioned values as endpoints. The protease hydrolysis time may be 3 hours, 4 hours, 5 hours, 6 hours, etc., or a value within a range of values having any two of the above-mentioned values as endpoints.

Based on the characteristics of high-lactic acid industrial by-products, the inventor has conducted rigorous research and found that adding nutrients to high-lactic acid industrial by-products, and further designing the formula, can promote yeast growth, and improve fermentation efficiency and yeast product quality. In an embodiment of the invention, the fermentation culture medium further comprises nutrients, wherein the nutrients comprise trace elements and vitamins. The trace element is selected from one or two or more of the group consisting of copper, calcium, iron, zinc, magnesium, and potassium, preferably copper and/or calcium; preferably, the vitamin is selected from one or two or more of the group consisting of vitamin B1, vitamin B2, vitamin B3, vitamin B6, pantothenic acid, and vitamin B7, preferably one or two or more of the group consisting of vitamin B1, pantothenic acid, and vitamin B7, more preferably vitamin B1, pantothenic acid, and vitamin B7.

In some embodiments of the present application, the nutrient comprises one or two or more of the group consisting of copper 2-5 ppm, calcium 400-1000 ppm, vitamin B1 10-30 ppm, pantothenic acid 15-30 ppm, and vitamin B7 0.5-1.5 ppm, preferably copper 2-5 ppm, calcium 400-1000 ppm, vitamin B1 10-30 ppm, pantothenic acid 15-30 ppm, and vitamin B7 0.5-1.5 ppm, based on the total weight of the fermentation culture medium.

For example, the mass content of copper, based on the total weight of the fermentation culture medium, may be 2 ppm, 3 ppm, 4 ppm, 5 ppm, etc., or a value within a range of values having any two of the above-mentioned values as endpoints; the mass content of calcium may be 400 ppm, 500 ppm, 600 ppm, 700 ppm, 800 ppm, 900 ppm, 1000 ppm, etc., or a value within a range of values having any two of the above-mentioned values as endpoints; the mass content of vitamin B1 may be 10 ppm, 15 ppm, 20 ppm, 25 ppm, 30 ppm, etc., or a value within a range of values having any two of the above-mentioned values as endpoints; the mass content of pantothenic acid may be 15 ppm, 20 ppm, 25 ppm, 30 ppm, etc., or a value within a range of values having any two of the above-mentioned values as endpoints; the mass content of vitamin B7 may be 0.5 ppm, 0.7 ppm, 1.0 ppm, 1.2 ppm, 1.5 ppm, etc., or a value within a range of values having any two of the above-mentioned values as endpoints.

In some embodiments of the present application, in order to further purify the yeast product, further included is a step of washing and/or drying the heavy phase obtained by centrifugation in step (4) with pure water.

Note that the strain activation culture medium, the primary seed culture medium, and the secondary seed culture medium of the present invention, and the culture conditions are not particularly limited as long as the culture medium and the culture conditions well known in the art are suitable for the growth of yeast strains.

In some embodiments of the present application, the primary seed culture medium in step (1) is a YPD liquid medium; preferably, the fermentation culture temperature is 25-35°C; preferably, the fermentation culture time is 12-30 hours; more preferably, the stirring speed is 150-220 rpm.

In some embodiments of the present application, the secondary seed culture medium in step (2) comprises glucose 5-8%, yeast leachate 0.3-0.6%, ammonium sulfate 1-2%, zinc sulfate 0.2-0.4%, potassium dihydrogen phosphate 0.4-0.6%, and magnesium sulfate 0.4-0.6% by mass volume percentage. Preferably, the fermentation culture temperature is 25-35°C; preferably the culture time is 12-36 hours; more preferably, the fermentation culture pH is 4.5-5.5; preferably the aeration is 2-4 L/min.

In a second aspect, the present application also provides a yeast produced by the above method, comprising 30-68%, preferably 50-68% of protein; preferably the yeast product comprises 1.5-9.0% of trehalose; more preferably, the yeast product contains 2.0-4.0% of phosphorus pentoxide.

Note that the form of the yeast product of the present invention is not particularly limited and may be, for example, a liquid, powder, or paste.

In a third aspect, the present application also provides the use of the yeast product as described above in a feed protein, food seasoning, or industrial fermentation nitrogen source. Such industrial fermentation nitrogen sources include, but are not limited to, yeast leachate.

Specifically, the above-mentioned yeast product can be directly used as a nitrogen source for industrial fermentation, and can also be further processed into yeast leachate. It should be understood that the yeast leachate refers to a powdery, pasty, or liquid product obtained by using fresh yeast as a raw material, using modern biological technical means or by changing the medium environment to induce various enzyme systems in the yeast itself, adding (not adding) some exogenous enzymes under appropriate conditions to promote the degradation of intracellular proteins and nucleic acid substances, and then performing some refining internal processes.

The above yeast products can also be used as high-quality protein raw materials for feed protein, which can increase the daily feed intake, average daily weight gain, and feed-to-weight ratio of animals.

The yeast product may be further processed into a yeast extract for use in the field of food seasoning to increase the delicious flavor of food.

The beneficial effects of the method for producing a yeast product using a high-lactic acid industrial by-product described herein are further illustrated by the following specific examples. The raw materials and equipment sources used in the examples and comparative examples of the present application are shown in Table 1.

Table 1 Raw materials used in inventive and comparative examples

| Raw materials or equipment used | Model/Purity | Vendor |
|---|---|---|
| Corn steep liquor | | Baolingbao Biological Co. Ltd. |
| Baijiu yellow water | | Anhui Gujinggongjiu Co. Ltd. |
| DDGS | | SDIC Bioenergy Tielin Co. Ltd. |
| Pickling water | | Sichuan Chuannan Food Factory |
| Cellulase | 350,000 U/mL | Angel Yeast Co. Ltd. |
| Protease | 600,000 U/mL | Angel Yeast Co. Ltd. |
| YPD culture medium | Reagent grade | Angel Yeast Co. Ltd. |
| Yeast leachate FM888 | Reagent grade | Angel Yeast Co. Ltd. |
| Magnesium sulfate heptahydrate | Analytical pure | Sinopharm Chemical Reagent Co., Ltd. |
| Zinc sulfate heptahydrate | Analytical pure | Sinopharm Chemical Reagent Co., Ltd. |
| *Candida tropicalis* | CGMCC2.0588 | China General Microbiological Culture Collection Center |
| *Streptomyces avermitilis* | ACCC 40168 | Agricultural Culture Collection of China |
| *Escherichia coli* | CICC 23933 | China Center of Industrial Culture Collection |

**Example 1**

Screening, identification method, and identification result of *Kluyveromyces marxianus*

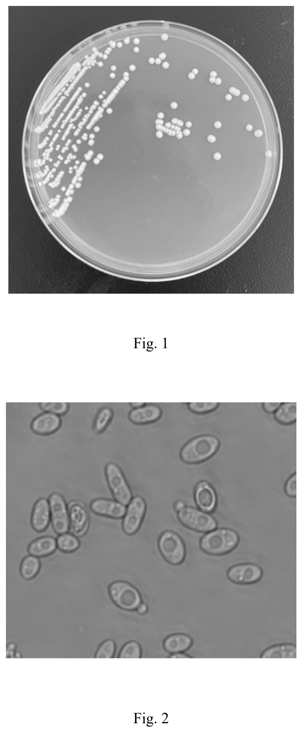

The samples of fermented dairy products collected in the Xizang region were dissolved into sterile water to mix well. The suspension was pipetted to prepare $10^{-5}$ and $10^{-6}$ suspensions by serial dilution of 10 times. The suspension was spread on a YPD culture medium, incubated at 30°C for 24-48 hours, and prepared to slides. The morphology of yeast was observed under a microscope, and the results are shown in Fig. 1. The characteristics of single colonies on the plate were observed at the same time, and the results are shown in Fig. 2. It can be seen from Figs. 1 and 2 that the colony of this yeast strain has a cheese-like texture, a milky white color, a smooth surface, neat edges, and an elliptical microscopic morphology, with budding reproduction. They were streaked twice for purification. The purified yeast strain was plated on a YPD slant medium and stored at 4°C. The genome of the yeast strain was extracted.

The yeast ITS sequences were amplified with ITS1 (5'-TCCGTAGGTGAACCTGCGG- 3', SEQ ID NO: 1) and ITS4 (5'-TCCTCCGCTTATTGATATGC- 3', SEQ ID NO: 2) as primers. After detection by 1% gel electrophoresis and sequencing, the yeast ITS sequences were aligned with the sequences on GenBank by Blast analysis. Sequence similarity greater than 99% indicates the same species. Based on morphological analysis and molecular identification, the strain was *Kluyveromyces marxianus,* with strain number AMCC30634. AMCC stands for Angel Microbial Culture Collection Center. The ITS sequence is shown in SEQ ID NO: 3. The strain was deposited at the China Type Culture Collection Center on February 27, 2023, with deposit number CCTCC NO: M 2023217.

Mutation of *Kluyveromyces marxianus*

The strain AMCC 30634 obtained above was inoculated into a test tube filled with 5 mL of YPD liquid at 2% inoculation volume and incubated at 30°C, 180 rpm for 20 hours. The yeast cell were collected by using a centrifuge, washed once with ddH$_2$O, and resuspended to a viable count of about 5 x 10$^7$ CFU/mL. 10 $\mu$L of suspension was pipetted dropped onto an iron plate and spread well. The mutagenesis conditions included: a treatment distance of 2 mm, a treatment power of 120 W, a gas flow rate of 10 SLM, a spotting amount of 10 $\mu$L, irradiation time of 0, 5, 10, 15, 20, 25, and 30 s respectively. Then, the samples were put into 1 mL of normal saline and mixed well. After gradient dilution, 0.1 mL of suspension was pipetted and spread on a YPD solid medium and incubated at 30°C for 48 hours. The lethality rate was determined according to the conventional method in the art. 98 colonies were selected from the plates with a lethality rate of > 98%. The single colony with a size superior to the starting strain was inoculated into a YPD culture medium for activation. 98 strains were all mutagenic strains, and one strain was obtained after screening and named AMCC 31342, which was deposited in the China Collection of Type Cultures on December 30, 2022, with deposit number CCTCC NO: M 20222110.

This example is a method for producing yeast using corn steep liquor comprising the following steps.

(1) Strain activation: the strain AMCC 31342 *(Kluyveromyces marxianus,* deposit number CCTCC NO: M20222110) was inoculated into a YPD solid medium for activation at an activation time of 30°C for an activation time of 60 hours to obtain purified strains. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Kluyveromyces marxianus* colonies were picked from the plate and inoculated in a YPD liquid medium, at an inoculation volume of 1% (v/v). The culture was carried out under shaking on a shaking table for 24 hours, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) Corn steep liquor fermentation: 25 L of corn steep liquor solution was prepared at a volume ratio of 2: 1 of corn steep liquor to water. The solution was added to a 50 L fermenter. After sterilization, the secondary seed liquid was inoculated into the fermenter at an inoculation volume of 6% (v/v). Under the control of a stirring speed of 400 rpm, an aeration rate of 30 L/min, a temperature of 28°C, and a pH of naturally increasing, the culture was carried out for 36 hours. In the corn steep liquor, the content of lactic acid was 12.3 wt% and the protein content was 22.7 wt%.

(5) A part of the fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product, which was designated as Example 1-1. The remaining fermentation broth after the fermentation in step (4) was subjected to continuous spray drying to obtain a yeast product, which was designated as Example 1-2.

The effect of yeast fermentation and the performance of yeast were measured as follows, and the results are shown in Table 3.

1) Wet weight determination

10 mL of fermentation broth after the fermentation was added to a 10 mL centrifuge tube which had been weighed and centrifuged at 5000 rpm for 10 min. The supernatant was discarded, and the remaining supernatant was gently spun away. The product was weighed and recorded as $m_1$, and calculated according to the formula below.

$$M = (m_1 - m_0) * 100$$

where: M-- yeast wet weight (g/L)
$m_1$--weight after centrifugation to discard the supernatant (g)
$m_0$--weight of the centrifuge tube (g)

2) Determination of protein

The Kjeldahl method specified in 6.4 of national standard GB/T 23530-2009 was adopted. A sample (equivalent to 30-440 mg of total nitrogen) was taken and added with 20 mL of concentrated sulfuric acid to perform digestion under the action of 5 g of a mixed catalyst (potassium sulfate and copper sulfate pentahydrate mixed in a ratio of 97: 3) and 2.5 g of a catalyst b (selenium powder and potassium sulfate mixed in a ratio of 0.1: 100), followed by distillation. The product ammonia was absorbed with boric acid and titrated with 0.1 mol/L hydrochloric acid. The data was read, and the total nitrogen content was calculated.

$$\text{Protein content} = \text{total nitrogen} * 6.25$$

3) Determination of phosphorus pentoxide

3.1 Principle

Phosphorus in the yeast is digested to produce water-soluble inorganic phosphorus, which reacts with ammonium molybdate to produce ammonium phosphomolybdate, which can produce molybdenum blue with a reducing agent under acidic conditions. Its color is directly proportional to the content of $P_2O_5$, which complies with Lambert-Beer law. The absorbance is measured at 700 nm with a spectrophotometer.

3.2 Main equipment: spectrophotometer, 5 mL graduated pipette, 100 mL volumetric flask

3.3 Reagents and solutions

6% ammonium molybdate solution: 6 g of ammonium molybdate [$(NH_4)6Mo_7O_{24}. 4H_2O$] was weighed, dissolved with water, diluted to 100 mL, and mixed well. It is noted that ammonium molybdate decomposes gradually during standing to produce water-insoluble $MoO_3$. A small amount of precipitation does not affect its use, but it is recommended not to prepare too much at once and to use it up within one month.
0.2% Metol solution: 0.2 g of Metol was weighed, dissolved with water, diluted to 100 mL, and mixed well.
1% sodium sulfite solution: 1 g of sodium sulfite was weighed, dissolved with water, diluted to 100 mL, and mixed well.
30% sodium pyrosulfate solution: 30 g of sodium pyrosulfate was weighed, dissolved with water, diluted to 100 mL, and mixed well.
Reducing agent solution: the above-mentioned 0.2% Metol solution, 1% sodium sulfite solution, and 30% sodium pyrosulfate solution were mixed in a ratio of 1: 1: 1 and stored in a brown reagent bottle.
Phosphorus standard solution: 0.191 g of reference potassium dihydrogen phosphate which had been dried at 105°C to constant weight was accurately weighed and dissolved with water. The solution was transferred into a 500 mL volumetric flask, added with water to scale and shaken well. 1 mL was equivalent to 0.2 mg of $P_2O_5$.

3.4 Procedures

Three 100 mL volumetric flasks were taken and respectively numbered according to blank, standard, and specimen. Then, the reagents were transferred into the 3 volumetric flasks in the order shown in Table 2 below.

Table 2 Steps for adding reagents

| Step | Blank | Standard | Specimen |
|---|---|---|---|
| (1) | / | Adding 3 mL of phosphorus standard solution | / |
| (2) | Adding 3 mL of 2.5 mol/L sulfuric acid | Same as the blank | / |
| (3) | Adding water to about 50 mL | Same as the blank | Same as the blank |
| (4) | Adding 3 mL of 6% ammonium molybdate | Same as the blank | Same as the blank |
| (5) | Adding 3 ml of reducing agent | Same as the blank | Same as the blank |
| (6) | Adding water to a constant volume of 100 mL | Same as the blank | Same as the blank |

The flasks were shaken well separately, and placed in a dark place for 1 hour. The blank was used as a control. The absorbance was measured at 700 nm using a spectrophotometer.

3.5 Result calculation

Calculation was performed according to the following formula:

$$X_7 = \frac{(E_1/E_2) \times 3 \times 0.2}{W \times Ds \times (5/100) \times 1000} \times 100\%$$

where, $X_7$--percent content of $P_2O_5$ in the specimen, %;
$E_1$--absorbance of the specimen;
$E_2$--absorbance of the $P_2O_5$ standard;
3--volume of the added $P_2O_5$ standard solution, mL;
0.2--concentration of $P_2O_5$ standard solution, g/L;
W--mass of the specimen, g;
Ds--percent content of accurate dry substance of the specimen, %;
5/100--Dilution factor of digestive juice.

The calculation results were retained to two decimal places.

4) Trehalose content determination

4.1 Principle

Anthrone reacts with trehalose to give the solution a bright green color in a proportional relationship. The absorbance is measured at 630 nm on a spectrophotometer to calculate the trehalose content.

4.2 Main equipment

a: analytical balance with a division value of 0.1 mg; b: spectrophotometer; c: centrifuge; d: oscillator; e: 10 mL graduated test tube; f: 5 mL graduated pipette; g: 1 mL graduated pipette; h: 50 mL volumetric flask; and i: 50 mL small beaker.

4.3 Reagents and solutions

a. Trichloroacetic acid: analytically pure, $CCl_3COOH=0.5$ mol/L; 40.8 g of trichloroacetic acid was weighed, dissolved with water, diluted to 500 mL, and mixed well.
b. Sulfuric acid: analytically pure, diluted sulfuric acid; to a 1000 mL beaker was added 150 mL of water. Then, 485 mL of sulfuric acid was taken by using a 500 mL measuring cylinder and slowly poured into the beaker, with gentle stirring while adding, followed by cooling.

c. Anthrone: analytically pure. 0.04 g of anthrone was weighed and added with 25 mL of diluted sulfuric acid (4.10.3.2) to be dissolved and mixed well.

4.4 Procedures

a. 0.15 g of well-mixed yeast milk was accurately weighed (accurate to 0.0002 g) and added to a centrifuge tube. 4.0 mL of 0.5 mol/L trichloroacetic acid solution was added. The mixture was shaken well on a shaker, placed in ice water with ice blocks, and shaken every 15 min for 1 hour.

b. Centrifuge was performed for 5 min by using a centrifuge at 3000 r/min.

c. The supernatant was poured into a 50 mL volumetric flask. The centrifuge tube and precipitate were washed with ice water and shaken to mix well. After that, the mixture was centrifuged by using a centrifuge at 3000 r/min for 5 min. The supernatant was poured into a 50 mL volumetric flask, diluted by using ice water to scale and shaken well.

d. Blank: to a 50 mL volumetric flask was added 4.0 mL of 0.5 mol/L trichloroacetic acid solution, which was then diluted to the scale with ice water and shaken well.

e. 1.00 mL of blank and sample were accurately pipetted to two clean and dry test tubes, then respectively accurately added with 5 mL of anthrone test solution and shaken well. The reaction was performed in a boiling water bath for 10 min. The tubes were immediately cooled to room temperature and shaken well. The absorbance was measured at 630 nm on a spectrophotometer using a 1 cm cuvette, with the blank as a control.

4.5 Result calculation

The content of trehalose in the specimen was calculated according to the following formula:

$$X_8 = \frac{E \times 6.29 \times 100}{W \times Ds \times 1000}$$

where: $X_8$--percent content of trehalose in the specimen, %;

E--absorbance of the specimen;

6.29--when E = 1, the mass of trehalose per milliliter of the solution, mg;

W--mass of the specimen, g;

Ds--percent content of accurate dry substance of specimen, %;

The calculation results were retained to two decimal places.

**Example 2**

Screening, identification method, and identification result of *Kluyveromyces lactis*

The samples of fermented dairy products collected in the Inner Mongolia region were dissolved into sterile water to mix well. The suspension was pipetted to prepare $10^{-5}$ and $10^{-6}$ suspensions by serial dilution of 10 times. The suspension was spread on a YPD culture medium, incubated at 30°C for 24-48 h, and prepared to slides. The morphology of yeast was observed under a microscope. The characteristics of single colonies on the plate were observed at the same time. The colony of this yeast strain has a cheese-like texture, a milky white color, a smooth surface, neat edges, and an elliptical microscopic morphology, with budding reproduction. They were streaked twice for purification. The purified yeast strain was plated on a YPD slant medium and stored at 4°C. The genome of yeast strains was extracted. The yeast ITS sequences were amplified with ITS1 (5'-TCCGTAGGTGAACCTGCGG- 3', SEQ ID NO: 1) and ITS4 (5'-TCC TCCGC TTATTGATATGC- 3', SEQ ID NO: 2) as primers. After detection by 1% gel electrophoresis and sequencing, the yeast ITS sequences were aligned with the sequences on GenBank by Blast analysis. Sequence similarity greater than 99% indicates the same species. Based on morphological analysis and molecular identification, the strain was *Kluyveromyces lactis,* with strain number AMCC31347. AMCC stands for Angel Microbial Culture Collection Center. The ITS sequence is shown in SEQ ID NO: 4. The strain was deposited at the China Type Culture Collection Center on February 27, 2023, with deposit number CCTCC NO M 2023218.

This example is a method for producing yeast using white Baijiu yellow water, including the following steps.

(1) Strain activation: the strain AMCC31347 was inoculated into a YPD solid medium for activation at 30°C for 60 hours to obtain the purified strain. The YPD solid medium was formulated as in Example 1.

(2) Primary seed liquid: 100 mL of YPD liquid medium was prepared and sterilized for later use. *Kluyveromyces lactis* colonies were picked from the plate and inoculated in a YPD liquid medium, at an inoculation volume of 2% (v/v). The culture was carried out under shaking on a shaking table for 30 hours, with the rotation speed of the shaking table being 220 rpm and the temperature being 33°C. The YPD liquid medium was formulated as in Example 1.

(3) Secondary seed liquid: 2 L of seed culture medium was prepared according to the following formula: according to mass volume percentage, glucose 8%, yeast leachate 0.6%, ammonium sulfate 2%, zinc sulfate 0.4%, potassium dihydrogen phosphate 0.6%, magnesium sulfate 0.6%, and a balance of deionized water. The prepared seed culture medium was added to a 3 L fermenter. After sterilization, the primary seed liquid was inoculated into the fermenter at an inoculation volume of 2% (v/v). Under the control of a stirring speed of 600 rpm, an aeration rate of 4 L/min, a pH of 5.5, and a temperature of 33°C, the culture was carried out for 36 hours.

(4) Baijiu yellow water fermentation: 35 L of Baijiu yellow water solution was prepared at a volume ratio of 1: 1 of Baijiu yellow water to water. The solution was added to a 50 L fermenter and added with 35 g of $(NH_4)_2HPO_3$. After sterilization, the secondary seed liquid was inoculated into the fermenter at an inoculation volume of 5% (v/v). Under the control of a stirring speed of 600 rpm, an aeration rate of 50 L/min, a temperature of 33°C, and a pH of naturally increasing, the culture was carried out for 48 hours. In the Baijiu yellow water, the lactic acid content was 11.5 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 3**

This example is a method for producing yeast using corn steep liquor comprising the following steps.

(1) Strain activation: the strain *Pichia kudriavzeii* C4.12 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Pichia kudriavzeii* colonies were picked from the plate and inoculated in a YPD liquid medium, at an inoculation volume of 1% (v/v). The culture was carried out under shaking on a shaking table for 24 h, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) Corn steep liquor fermentation: 25 L of corn steep liquor solution was prepared at a volume ratio of 2: 1 of corn steep liquor to water. The solution was added to a 50 L fermenter. After sterilization, the secondary seed liquid was inoculated into the fermenter at an inoculation volume of 6% (v/v). Under the control of a stirring speed of 400 rpm, an aeration rate of 30 L/min, a temperature of 28°C, and a pH of naturally increasing, the culture was carried out for 36 hours. In the corn steep liquor, the content of lactic acid was 12.3 wt% and the protein content was 22.7 wt%.

(5) The fermentation broth after the fermentation in step (4) was subjected to 5000 rpm centrifugation for 10 minutes to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 4**

This example is a method for producing yeast using DDGS including the following steps.

(1) Strain activation: the strain *Candida tropicalis* CGMCC 2.0588 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Candida tropicalis* colonies were picked from the plate and inoculated in a YPD liquid medium, at an inoculation volume of 1% (v/v). The culture was carried out under shaking on a shaking table for 24 h, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) DDGS fermentation: 35 L of DDGS solution was prepared at a mass volume ratio of 1:10 of DDGS to water. The solution was added to a 50 L air-lift fermenter. After sterilization, the secondary seed liquid was inoculated into the fermenter at an inoculation volume of 5% (v/v). Under an aeration rate of 350 L/min, a temperature of 33°C, and a pH of naturally increasing, the culture was carried out for 48 hours. In the DDGS, the lactic acid content was 10.3 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 5**

This example is a method for producing yeast using corn steep liquor comprising the following steps.

(1) Strain activation: the strain AMCC 31342 *(Kluyveromyces marxianus,* deposit number CCTCC No. M 20222110) of Example 1 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Kluyveromyces marxianus* colonies were picked from the plate and inoculated in a YPD liquid medium, at an inoculation volume of 1% (v/v). The culture was carried out under shaking on a shaking table for 24 h, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) Corn steep liquor fermentation: 25 L of corn steep liquor solution was prepared at a volume ratio of 2: 1 of corn steep liquor to water. The solution was added to a 50 L fermenter. After sterilization of the fermentation medium, the secondary seed liquid was inoculated into the fermenter at an inoculation volume of 6% (v/v). To the fermenter were added copper sulfate, calcium carbonate, vitamin B1, pantothenic acid, and vitamin B7, wherein based on the total weight of the fermenter medium, the mass content of copper was 5 ppm; the mass content of calcium was 400 ppm; the mass content of vitamin B1 was 10 ppm; the mass content of pantothenic acid was 15 ppm; the mass content of vitamin B7 was 0.5 ppm. Under the control of a stirring speed of 400 rpm, an aeration rate of 30 L/min, a temperature of 28°C, and a pH of naturally increasing, the culture was carried out for 36 hours. In the corn steep liquor, the content of lactic acid was 12.3 wt% and the protein content was 22.7 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 6**

This example is a method for producing yeast using white Baijiu yellow water, including the following steps.

(1) Strain activation: strain AMCC31347 *(Kluyveromyces lactis,* deposit number CCTCC NO: M2023218) of Example 2 was inoculated into a YPD solid medium for activation at 30°C for 60 hours to obtain a purified strain. The YPD solid medium was formulated as in Example 1.

(2) Primary seed liquid: 100 mL of YPD liquid medium was prepared and sterilized for later use. *Kluyveromyces lactis* colonies were picked from the plate and inoculated in a YPD liquid medium, at an inoculation volume of 2% (v/v). The culture was carried out under shaking on a shaking table for 30 hours, with the rotation speed of the shaking table being 220 rpm and the temperature being 33°C. The YPD liquid medium was formulated as in Example 1.

(3) Secondary seed liquid: 2 L of seed culture medium was prepared according to the following formula: according to mass volume percentage, glucose 8%, yeast leachate 0.6%, ammonium sulfate 2%, zinc sulfate 0.4%, potassium dihydrogen phosphate 0.6%, magnesium sulfate 0.6%, and a balance of deionized water. The prepared seed culture medium was added to a 3 L fermenter. After sterilization, the primary seed liquid was inoculated into the fermenter at an inoculation volume of 2% (v/v). Under the control of a stirring speed of 600 rpm, an aeration rate of 4 L/min, a pH of 5.5, and a temperature of 33°C, the culture was carried out for 36 hours.

(4) Baijiu yellow water fermentation: 35 L of Baijiu yellow water solution was prepared at a volume ratio of 1: 1 of Baijiu yellow water to water. The solution was added to a 50 L fermenter as well as 35 g of $(NH_4)_2HPO_3$. After sterilization of the fermentation medium, the secondary seed liquid was inoculated into the fermenter at an inoculation volume of 5% (v/v). To the fermenter were added copper sulfate, calcium carbonate, vitamin B1, pantothenic acid, and vitamin B7, wherein based on the total weight of the fermenter medium, the mass content of copper was 2 ppm; the mass content of calcium was 600 ppm; the mass content of vitamin B1 was 30 ppm; the mass content of pantothenic acid was 20 ppm; the mass content of vitamin B7 was 1 ppm. Under the control of a stirring speed of 600 rpm, an aeration rate of 50 L/min, a temperature of 33°C, and a pH of naturally increasing, the culture was carried out for 48 hours. In the Baijiu yellow water, the lactic acid content was 11.5 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 7**

This example is a method for producing yeast using corn steep liquor comprising the following steps.

(1) Strain activation: the strain *Pichia kudriavzeii* C4.12 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Pichia kudriavzeii* colonies were picked from the plate and inoculated in a YPD liquid medium, at an inoculation volume of 1% (v/v). The culture was carried out under shaking on a shaking table for 24 h, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) Corn steep liquor fermentation: 25 L of corn steep liquor solution was prepared at a volume ratio of 2: 1 of corn steep liquor to water. The solution was added to a 50 L fermenter. After sterilization, the secondary seed liquid was inoculated into the fermenter at an inoculation volume of 6% (v/v). To the fermenter were added copper sulfate, calcium carbonate, vitamin B1, pantothenic acid, and vitamin B7, wherein based on the total weight of the fermenter medium, the mass content of copper was 3 ppm; the mass content of calcium was 1000 ppm; the mass content of vitamin B1 was 20 ppm; the mass content of pantothenic acid was 30 ppm; the mass content of vitamin B7 was 1.5 ppm. Under the control of a stirring speed of 400 rpm, an aeration rate of 30 L/min, a temperature of 28°C, and a pH of naturally increasing, the culture was carried out for 36 hours. In the corn steep liquor, the content of lactic acid was 12.3 wt% and the protein content was 22.7 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 8**

This example is a method for producing yeast using DDGS including the following steps.

(1) Strain activation: the strain *Candida tropicalis* CGMCC 2.0588 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Candida tropicalis* colonies were picked from the plate and inoculated in a YPD liquid medium, at an inoculation volume of 1% (v/v). The culture was carried out under shaking on a shaking table for 24 h, with the rotation speed of the shaking table being 180 rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) DDGS fermentation: 35 L of DDGS solution was prepared at a mass volume ratio 1: 10 of DDGS to water. The solution was added to a 50 L air-lift fermenter. After sterilization, the secondary seed liquid was inoculated into the fermenter at an inoculation volume of 5% (v/v). To the fermenter were added copper sulfate, calcium carbonate, vitamin B1, pantothenic acid, and vitamin B7, wherein based on the total weight of the fermenter medium, the mass content of copper was 3 ppm; the mass content of calcium was 600 ppm; the mass content of vitamin B1 was 15 ppm; the mass content of pantothenic acid was 20 ppm; the mass content of vitamin B7 was 0.75 ppm. Under an aeration rate of 350 L/min, a temperature of 33°C, and a pH of naturally increasing, the culture was carried out for 48 hours. In the DDGS, the lactic acid content was 10.3 wt%.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 9**

This example is a method for producing yeast using DDGS including the following steps.

(1) Strain activation: strain AMCC31347 *(Kluyveromyces lactis,* deposit number CCTCC NO: M2023218) of Example 2 was inoculated into a YPD solid medium for activation at 30°C for 60 hours to obtain a purified strain. The YPD solid medium was formulated as in Example 1.

(2) Primary seed liquid: 100 mL of liquid YPD culture medium was prepared and sterilized for later use. *Kluyveromyces lactis* colonies were picked from the plate and inoculated in a YPD liquid medium, at an inoculation volume of 1% (v/v). The culture was carried out under shaking on a shaking table for 30 hours, with the rotation speed of the shaking table being 220 rpm and the temperature being 33°C. The liquid YPD culture medium was formulated as in Example 1.

(3) Secondary seed liquid: 2 L of seed culture medium was prepared according to the following formula: according to mass volume percentage, glucose 6%, yeast leachate 0.6%, ammonium sulfate 2%, zinc sulfate 0.4%, potassium dihydrogen phosphate 0.6%, magnesium sulfate 0.6%, and a balance of deionized water. The prepared seed culture medium was added to a 3 L fermenter. After sterilization, the primary seed liquid was inoculated into the fermenter at an inoculation volume of 3% (v/v). Under the control of a stirring speed of 600 rpm, an aeration rate of 4 L/min, a pH of 5.8, and a temperature of 33°C, the culture was carried out for 36 hours.

(4) DDGS fermentation: 35 L of DDGS solution was prepared at a mass volume ratio of 1:10 of DDGS to water. The solution was added to a 50 L fermenter. After sterilization, the secondary seed liquid was inoculated into the fermenter at an inoculation volume of 5% (v/v). To the fermenter were added copper sulfate, calcium carbonate, vitamin B1, pantothenic acid, and vitamin B7 to prepare a fermenter medium, wherein based on the total weight of the fermenter medium, the mass content of copper was 3 ppm; the mass content of calcium was 600 ppm; the mass content of vitamin B1 was 15 ppm; the mass content of pantothenic acid was 20 ppm; the mass content of vitamin B7 was 0.75 ppm. Under the control of a stirring speed of 600 rpm, an aeration rate of 50 L/min, a temperature of 33°C, and a pH of naturally increasing, the culture was carried out for 48 hours.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 10**

This example is a method for producing yeast using DDGS including the following steps.

(1) Strain activation: strain AMCC31347 *(Kluyveromyces lactis,* deposit number CCTCC NO: M2023218) of Example 2 was inoculated into a YPD solid medium for activation at 30° for 60 hours to obtain a purified strain. The YPD solid medium was formulated as in Example 1.

(2) Primary seed liquid: 100 mL of YPD liquid medium was prepared and sterilized for later use. *Kluyveromyces lactis* colonies were picked from the plate and inoculated in a YPD liquid medium, at an inoculation volume of 1% (v/v). The culture was carried out under shaking on a shaking table for 30 hours, with the rotation speed of the shaking table being 220 rpm and the temperature being 33°C. The YPD liquid medium was formulated as in Example 1.

(3) Secondary seed liquid: 2 L of seed culture medium was prepared according to the following formula: according to mass volume percentage, glucose 6%, yeast leachate 0.6%, ammonium sulfate 2%, zinc sulfate 0.4%, potassium dihydrogen phosphate 0.6%, magnesium sulfate 0.6%, and a balance of deionized water. The prepared seed culture medium was added to a 3 L fermenter. After sterilization, the primary seed liquid was inoculated into a 3 L fermenter at an inoculation volume of 3% (v/v). Under the control of a stirring speed of 600 rpm, an aeration rate of 4 L/min, a pH of 5.8, and a temperature of 33°C, the culture was carried out for 36 hours.

(4) DDGS modification: DDGS and water were mixed at a mass-volume ratio of 1: 10 to prepare 40 L of DDGS solution. The DDGS solution was then heated to 60°C, added with 4.0 wt%o of cellulase (according to the dry basis weight of the DDGS solution), and subjected to enzymolysis for 5 hours. Then, 3 wt%o of protease (based on the dry weight of the DDGS solution) was added for enzymolysis for 4 hours. Finally, the solution was heated up to 75°C and subjected to inactivation for 30 min to obtain modified DDGS. In the DDGS, the lactic acid content was 10.3 wt%.

(5) To a 50 L of fermenter was added 35 L of the modified DDGS. After sterilization, the secondary seed liquid was inoculated into the fermenter at an inoculation volume of 5% (v/v). To the fermenter were added copper sulfate, calcium carbonate, vitamin B1, pantothenic acid, and vitamin B7, wherein based on the total weight of the fermenter medium, the mass content of copper was 3 ppm; the mass content of calcium was 600 ppm; the mass content of vitamin B1 was 15 ppm; the mass content of pantothenic acid was 20 ppm; the mass content of vitamin B7 was 0.75 ppm. Under the control of a stirring speed of 600 rpm, an aeration rate of 50 L/min, a temperature of 33°C, and a pH of naturally increasing, the culture was carried out for 48 hours.

(6) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 11**

The strain AMCC 31342 *(Kluyveromyces marxianus,* accession No. CCTCC No: M 20222110) of Example 1 was subjected to strain activation, primary seed culture, and secondary seed culture according to the method of Example 5 to obtain a secondary seed culture broth for later use.

Corn steep liquor modification: corn steep liquor and water were mixed at a mass-volume ratio of 2: 1 to prepare 30 L of corn steep liquor solution. The corn steep liquor solution was then heated to 55°C, added with 6 wt‰ of cellulase (according to the dry basis weight of the corn steep liquor solution), and subjected to enzymolysis for 6 hours. Then, 2 wt‰ of protease (based on the dry weight of the corn steep liquor) was added for enzymolysis for 3 hours. Finally, the solution was heated up to 70°C and subjected to inactivation for 40 min to obtain modified corn steep liquor. In the corn steep liquor, the content of lactic acid was 12.3 wt% and the protein content was 22.7 wt%.

Modified corn steep liquor fermentation: 25 L of the corn steep liquor solution above was added to a 50 L fermenter. After sterilization of the fermentation medium, the prepared secondary seed culture broth was inoculated into the fermenter at an inoculation volume of 6% (v/v). To the fermenter were added copper sulfate, calcium carbonate, vitamin B1, pantothenic acid, and vitamin B7, wherein based on the total weight of the fermenter medium, the mass content of copper was 5 ppm; the mass content of calcium was 400 ppm; the mass content of vitamin B1 was 10 ppm; the mass content of pantothenic acid was 15 ppm; the mass content of vitamin B7 was 0.5 ppm. Under the control of a stirring speed of 400 rpm, an aeration rate of 30 L/min, a temperature of 28°C, and a pH of naturally increasing, the culture was carried out for 36 hours.

The fermentation broth obtained after the fermentation was subjected to 5000 rpm centrifugation for 10 minutes to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 12**

The strain AMCC31347 *(Kluyveromyces lactis,* accession No. CCTCC NO: M2023218) of Example 2 was subjected to strain activation, primary seed culture, and secondary seed culture according to the method of Example 6 to obtain a secondary seed culture broth for later use.

Baijiu yellow water modification: Baijiu yellow water and water were mixed at a mass-volume ratio of 1: 1 to prepare 40 L of Baijiu yellow water solution. The Baijiu yellow water solution was then heated to 65°C, added with 3 wt‰ of cellulase (according to the dry basis weight of the Baijiu yellow water solution), and subjected to enzymolysis for 3 hours. Then, 4 wt‰ of protease (based on the dry weight of the Baijiu yellow water) was added for enzymolysis for 6 hours. Finally, the solution was heated up to 90°C and subjected to inactivation for 20 min to obtain modified Baijiu yellow water. In the Baijiu yellow water, the lactic acid content was 11.5 wt%. Modified Baijiu yellow water fermentation: 35 L of modified Baijiu yellow water solution was added to a 50 L fermenter as well as 35 g of $(NH_4)_2HPO_3$. After sterilization of the fermentation medium, the prepared secondary seed culture broth was inoculated into the fermenter at an inoculation volume of 5% (v/v). To the fermenter were added copper sulfate, calcium carbonate, vitamin B1, pantothenic acid, and vitamin B7, wherein based on the total weight of the fermenter medium, the mass content of copper was 2 ppm; the mass content of calcium was 600 ppm; the mass content of vitamin B1 was 30 ppm; the mass content of pantothenic acid was 20 ppm; the mass content of vitamin B7 was 1 ppm. Under the control of a stirring speed of 600 rpm, an aeration rate of 50 L/min, a temperature of 33°C, and a pH of naturally increasing, the culture was carried out for 48 hours.

The fermentation broth obtained after the fermentation was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Example 13**

The differences from Example 5 were that in step (4) the stirring speed was 300 rpm and the aeration rate was 12.5 L/min.

The effect of yeast fermentation and the performance of yeast were measured as in Example 1, and the results are shown in Table 3.

**Example 14**

The differences from Example 6 were that in step (4) the stirring speed was 700 rpm and the aeration rate was 75 L/min.

The effect of yeast fermentation and the performance of yeast were measured as in Example 1, and the results are shown in Table 3.

**Example 15**

Compared to Example 5, the differences were that no vitamin B1, pantothenic acid, and vitamin B7 were added to the corn steep liquor fermentation in step (4).

**Example 16**

Compared to Example 5, the differences were that no copper sulfate and calcium carbonate were added to the corn steep liquor fermentation in step (4).

**Comparative Example 1**

The difference from Example 1 was that Comparative Example 1 adopted *Saccharomyces cerevisiae* FX-2 as the fermentation strain. The others were the same as Example 1.

In step (5), a part of the fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product, which was designated as Comparative Example 1-1. The remaining fermentation broth after the fermentation in step (4) was subjected to continuous spray drying to obtain a yeast product, which was designated as Comparative Example 1-2.

The effect of yeast fermentation and the performance of yeast were measured as in Example 1, and the results are shown in Table 3.

**Comparative Example 2**

The difference from Example 1 was that in step (4), molasses was used for fermentation. The specific method was as follows:

(1) Strain activation: the strain AMCC 31342 *(Kluyveromyces marxianus,* deposit number CCTCC NO: M20222110) of Example 1 was inoculated into a YPD solid medium for activation at an activation time of 30°C for an activation time of 60 hours to obtain a purified strain. The YPD solid medium was composed of 10 g of yeast extract, 20 g of peptone, 20 g of glucose, and 20 g of agar, added with distilled water to a constant volume of 1000 mL, and autoclaved at 121°C for 15 min.

(2) Primary seed liquid: 50 mL of YPD liquid medium was prepared and sterilized for later use. *Kluyveromyces marxianus* colonies were picked from the plate and inoculated in a YPD liquid medium, at an inoculation volume of 1% (v/v). The culture was carried out under shaking on a shaking table for 24 h, with the rotation speed of the shaking table being 180rpm and the temperature being 28°C.

The YPD liquid culture medium included: 1 wt% of yeast cream, 2 wt% of yeast peptone, 2 wt% of glucose, and a balance of deionized water.

(3) Secondary seed liquid: 1.5 L of seed culture medium was prepared according to the following formula: according to mass volume percentage, glucose 5%, yeast leachate 0.3%, ammonium sulfate 1%, zinc sulfate 0.2%, potassium dihydrogen phosphate 0.4%, magnesium sulfate 0.4%, and a balance of deionized water. The prepared seed culture medium was added to a 3 L fermenter. After sterilization, the primary seed liquid was inoculated into the 3 L fermenter at an inoculation volume of 3% (v/v). Under the control of a stirring speed of 400 rpm, an aeration rate of 2 L/min, a pH of 4.5, and a temperature of 28°C, the culture was carried out for 24 hours.

(4) Molasses fermentation: 25 L of corn steep liquor solution was prepared at a volume ratio of 2: 1 of molasses to water. The solution was added to a 50 L fermenter and added with 350 g of ammonium sulfate and 200 g of potassium dihydrogen phosphate. After sterilization, the secondary seed liquid was inoculated into the fermenter at an inoculation volume of 6% (v/v). Under the control of a stirring speed of 400 rpm, an aeration rate of 30 L/min, a temperature of 28°C, and a pH of naturally increasing, the culture was carried out for 36 hours.

(5) The fermentation broth after the fermentation in step (4) was subjected to 5000 rpm centrifugation for 10 min to collect heavy-phase yeast cell. Then the yeast cell were washed with pure water and subjected to repeated centrifugation to obtain a yeast product.

The effect of yeast fermentation and the performance of yeast were measured as in Example 1, and the results are shown in Table 3.

**Comparative Example 3**

The difference from Example 2 was that Comparative Example 2 adopted *Saccharomyces cerevisiae* FX-2 as the fermentation strain. The others were the same as Example 2.

The effect of yeast fermentation and the performance of yeast were measured as in Example 1, and the results are shown in Table 3.

Table 3 Assay Results

|  | Harvest wet weight | Protein | Phosphorus pentoxide | Trehalose |
|---|---|---|---|---|
| Example 1-1 | 183 g/L | 55.32% | 2.47% | 4.15% |
| Example 1-2 | 183 g/L | 35.71% | 2.47% | 3.34% |
| Example 2 | 154 g/L | 59.88% | 2.35% | 5.76% |
| Example 3 | 164 g/L | 63.21% | 2.66% | 2.17% |
| Example 4 | 165 g/L | 59.15% | 3.15% | 2.96% |
| Example 5 | 224 g/L | 54.85% | 2.55% | 3.51% |
| Example 6 | 224 g/L | 60.58% | 3.51% | 6.95% |
| Example 7 | 207 g/L | 67.33% | 2.37% | 1.81% |
| Example 8 | 211 g/L | 58.21% | 3.06% | 3.22% |
| Example 9 | 160 g/L | 53.61% | 2.11% | 8.42% |
| Example 10 | 208 g/L | 53.15% | 2.63% | 6.22% |
| Example 11 | 242 g/L | 53.17% | 2.48% | 3.61% |
| Example 12 | 237 g/L | 58.18% | 2.17% | 4.22% |
| Example 13 | 155 g/L | 56.15% | 2.62% | 4.27% |
| Example 14 | 226 g/L | 60.33% | 3.44% | 5.85% |
| Example 15 | 183 g/L | 53.35% | 2.62% | 2.88% |
| Example 16 | 181 g/L | 54.75% | 3.11% | 2.96% |
| Comparative Example 1-1 | 102 g/L | 43.14% | 2.65% | 5.62% |
| Comparative Example 1-2 | 102 g/L | 37.03% | 2.23% | 4.17% |
| Comparative Example 2 | 228 g/L | 53.77% | 2.49% | 4.53% |
| Comparative Example 3 | 69 g/L | 42.17% | 2.37% | 8.33% |

As can be seen from Table 3, the harvest wet weights of the yeast produced in Examples 1-16 of the present invention were 155-242 g/L; the protein contents in the yeast product after separation were 53.15-67.33%; the contents of phosphorus pentoxide were 2.11-3.51%; and the contents of trehalose were 1.81-8.42%, indicating that the yeast produced by the method of the present invention has stable fermentation process, high production efficiency and stable product quality. Compared with Example 1, in Comparative Example 1, fermentation with *Saccharomyces cerevisiae* resulted in a harvest wet weight of 102 g/L and a significant decrease in fermentation efficiency. The protein content in the yeast product after separation was 43.14%, which was significantly lower than that in Example 1. The yeast products obtained by direct spray drying (Examples 1-2 and Comparative Examples 1-2) had little difference in protein content, mainly due to the presence of unconverted protein in the fermentation substrate corn steep liquor. Compared with Example 2, comparative example 3 adopted Saccharomyces cerevisiae for fermentation, and the harvest wet weight was only 69 g/L, with significantly decreased fermentation efficiency; and the protein content in the separated yeast product was 42.17%, which was significantly lower than that in Example 2. It can be seen that using the specific yeast species of the present invention to ferment high-lactic acid industrial by-products has a higher fermentation efficiency, and the yeast product obtained has a higher yeast protein content.

Compared with Example 5, Comparative Example 2 adopted molasses as a carbon source for fermentation. The harvest wet weight was 228 g/L, showing that the fermentation efficiencies of Example 5 and Comparative Example 2 were comparable, but the costs of high-lactic acid industrial by-products such as corn steep liquor as an industrial waste are much lower than that of molasses.

Compared with Examples 1-4, the addition of nutrients in Examples 5-8 increased the harvest wet weight by 22.4%, 45.5%, 26.2%, and 27.9%, respectively, indicating that the nutrients could significantly promote the growth of yeast in the high-lactic acid industrial by-product fermentation substrate. Compared with Example 5, Example 15 only added copper and calcium, and the harvest wet weight was 183 g/L. Example 16 only added vitamin B1, pantothenic acid, and vitamin, and the harvest wet weight was 181 g/L. Example 1 neither added copper and calcium, nor added vitamin B1, pantothenic acid, and vitamin, and the harvest wet weight was 183 g/L. It can be seen that the simultaneous addition of trace elements and vitamins can achieve a synergistic effect in the process of producing yeast using a high-lactic acid industrial by-product.

Compared with Example 9, in Example 10, the DDGS was firstly treated by enzymolysis and then fermented, and the harvest wet weight was increased by 30%. Compared with Example 5, in Example 11, the DDGS was firstly treated by enzymolysis and then fermented, and the harvest wet weight increased by 8%. Compared with Example 6, in Example 12, the DDGS was firstly treated by enzymolysis and then fermented, the harvest wet weight increased by 5.8%. It can be seen that enzymatic modification of high-lactic acid industrial by-products before fermentation increases the content of sugars and amino acids in high-lactic acid industrial by-products, which can be better absorbed and transformed by yeast to improve fermentation efficiency.

Compared with Example 5, the rotation speed and aeration ratio in the fermentation culture stage of Example 13 were lower, so the fermentation broth has insufficient dissolved oxygen, affecting the growth of yeast, and finally leading to a lower wet weight. Compared with Example 5, the rotation speed and aeration ratio in the fermentation culture stage of Example 14 were higher, and the increase in the harvest wet weight was not obvious. However, bubble flooding easily occurred during the fermentation process, which increased the difficulty of production control.

## Experimental Example 1

The yeasts obtained in Examples 1-16 and Comparative Examples 1-3 (wherein the yeast products obtained after separation were used in both Example 1 and Comparative Example 1) were directly used as a nitrogen source for culturing *Streptomyces avermitilis* ACCC 40168 (an antibiotic fermenting strain). The specific method is as follows:

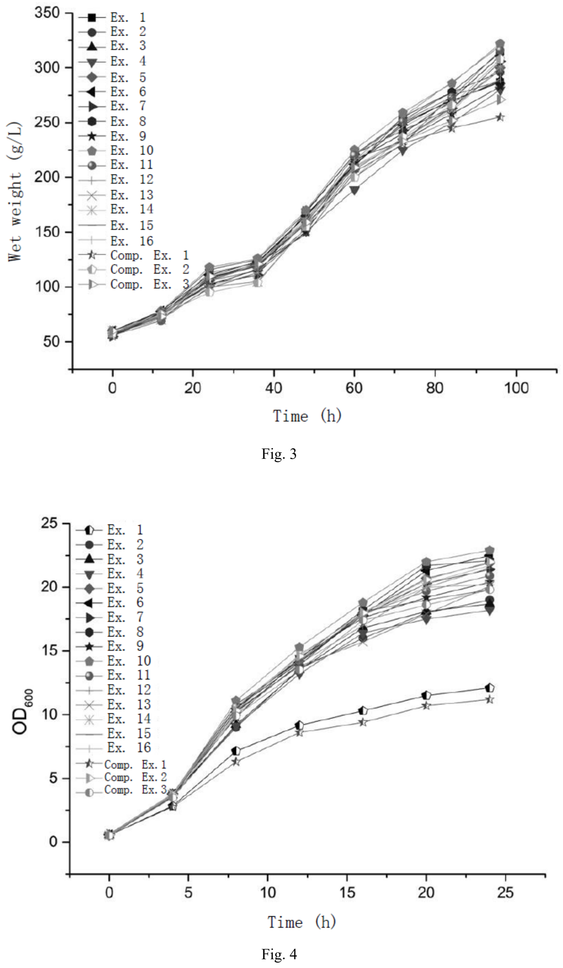

1. 100 mL of culture medium was prepared according to glucose 40 g/L, potassium dihydrogen phosphate 5 g/L, yeast (Examples 1-6, Comparative Examples 1-2, based on dry basis) 10 g/L, and put into a 500 mL conical flask for sterilization.
2. A spore suspension of *Streptomyces avermitilis* spores was prepared using sterile water. 5 mL of the suspension was pipetted to inoculate into the culture medium in Step 1 and subjected to shaking culture at 25°C, 180 rpm for 96 hours. During the culture, samples were taken for wet weight measurement every 12 hours.
3. The test results are shown in Fig. 3.

It can be seen from Fig. 3 that when the yeast product prepared by the present invention and the yeast product prepared by using molasses fermentation were used for fermenting a nitrogen source, the fermentation effects were equivalent; also, as the fermentation time was prolonged, the fermentation effect of the yeast product prepared in the present invention was significantly better than that of the yeast products prepared in Comparative Examples 1 and 3.

## Experimental Example 2

Yeast leachates were prepared by using the yeasts obtained in Examples 1-16 and Comparative Examples 1-3 (wherein the yeast products obtained after separation were used in both Example 1 and Comparative Example 1). Then, *Escherichia coli* CICC 23933 was cultured using the yeast leachates as nitrogen sources. The specific method for preparing the yeast leachate is as follows.

1. The yeast was added with water to prepare a yeast milk of 14% (w/w).
2. The pH of the yeast milk obtained in Step 1 was adjusted to 5.5-5.8. The temperature was maintained at 55°C for 20 hours, then increased to 70°C, and maintained for 30 min to inactivate the enzyme.
3. Centrifuge was carried out at 5000 rpm for 10 min and the supernatant was collected.
4. The supernatant was concentrated in a rotary evaporator and then spray-dried to a powdered yeast leachate.

The yeast leachates prepared in Examples 1-16 and Comparative Examples 1-3 were obtained by the above-mentioned method, respectively. Then, the culture test of *Escherichia coli* was performed as follows using the yeast leachates prepared in Examples 1-16 and Comparative Examples 1-3, respectively, as the nitrogen sources.

1. A 100 mL culture medium was prepared according to glucose 30 g/L, sodium chloride 5 g/L, any of the yeast leachates prepared in Examples 1-16 and Comparative Examples 1-3 15 g/L, filled into a 500 mL conical flask, and sterilized for later use.
2. A single colony of E. coli was picked from the slant, inoculated into the culture medium of step 1, and cultured at 37°C, 200 rpm for 24 hours with shaking. Samples were taken every 4 h during this period, and the $OD_{600}$ was measured with a spectrophotometer (i.e. the absorbance of the solution at 600 nm). The results are shown in Fig. 4.

It can be seen from Fig. 4 that the product quality of the yeast leachate prepared by using the yeast products of Examples 1-16 of the present invention as the organic nitrogen source was equivalent to that of the prior art yeast produced by using molasses (Comparative Example 2), indicating that the yeast produced by the method of the present invention can meet the market demand, and the industrial application of yeast for producing high-lactic acid industrial by-products can be realized, increasing the channels for obtaining yeast sugar resources, widening the application range of high-lactic acid industrial by-products, and alleviating the problem of shortage of molasses resources in the yeast industry.

The $OD_{600}$ of Comparative Example 1 and Comparative Example 3 were significantly lower than those of Examples 1 and 2, indicating that the densities of the yeasts in the fermentation broths were lower, i.e. the yeast product produced by the present invention using a specific strain can produce yeast leachate that acts as an organic nitrogen source for better fermentation.

**Experimental Example 3**

The yeast leachate prepared in the above Experimental Example 2 (Example 11, Comparative Example 2) was taken for sensory evaluation to investigate the difference in the mouthfeel of the yeast leachates prepared in the two schemes. The score was 0-10 according to the mouthfeel, with 0 being extremely poor and 10 being extremely excellent. The specific operations are:

2% aqueous solution sensory evaluation

The two yeast leachates were prepared as a 2% (w/v) hot aqueous solution (60°C), and the two samples were evaluated blindly by a sensory panel of 10 persons. Then, the mouthfeels were scored, and the results are shown in Table 4.

Table 4 Sensory evaluation results of yeast leachates obtained in Example 11 and Comparative Example 2

|  | I | II | III | IV | V | VI | VII | VIII | IX | X | Average |
|---|---|---|---|---|---|---|---|---|---|---|---|
| Ex. 11 | 7.5 | 8.5 | 7.5 | 8 | 7.5 | 8.5 | 8.5 | 7.5 | 8 | 8 | 7.95 ± **0.44** |
| Comp. Ex. 2 | 7.0 | 6.5 | 7 | 7.5 | 7.5 | 7 | 6.5 | 7 | 7.5 | 7 | 7.05 ± 0.37 |

As can be seen from Table 4, the yeast extract prepared in Example 11 had a higher mouthfeel score than the yeast extract prepared in Comparative Example 2, indicating that fermentation of yeast with a high-lactic acid industrial by-product, such as corn steep liquor, could produce more flavor and make the final product more palatable and advantageous in the field of flavoring. Finally, it should be noted that the preferred examples above are only used to illustrate the technical solution of the present invention and not to limit it. Although the present invention has been described in detail through the preferred embodiments described above, those skilled in the art should understand that various changes can be made in form and detail without departing from the scope of the claims of the present invention.

# EP 4 696 769 A1

SEQUENCE LISTING

| SEQ ID NO. | SEQ Descriptions | SEQ |
|---|---|---|
| SEQ ID NO: 1 | Primer SEQ | TCCGTAGGTGAACCTGCGG |
| SEQ ID NO: 2 | Primer SEQ | TCC TCCGCTTATTGATATGC |
| SEQ ID NO:3 | ITS SEQ of *Kluyveromyces marxianus* AMCC 30634 | CCAACGGGGATTGCCTTAGTACGGCGAGTGAAG CGGCAAAAGCTCAAATTTGAAATCTGGCGTCTTC GACGTCCGAGTTGTAATTTGAAGAAGGCGACTT TGTAGCTGGTCCTTGTCTATGTTCCTTGGAACAG GACGTCATAGAGGGTGAGAATCCCGTGTGGCGA GGATCCCAGTTATTTGTAAAGTGCTTTCGACGAG TCGAGTTGTTTGGGAATGCAGCTCTAAGTGGGTG GTAAATTCCATCTAAAGCTAAATATTGGCGAGA GACCGATAGCGAACAAGTACAGTGATGGAAAGA TGAAAAGAACTTTGAAAAGAGAGTGAAAAAGTA CGTGAAATTGTTGAAAGGGAAGGGCATTTGATC AGACATGGCGTTTGCTTCGGCTTTCGCTGGGCCA GCATCAGTTTTAGCGGTTGGATAAATCCTCGGGA ATGTGGCTCTGCTTCGGTAGAGTGTTATAGCCCG TGGGAATACAGCCAGCTGGGACTGAGGATTGCG ACTTTTGTCAAGGATGCTGGCGTAATGGTTAAAT GCCGCCCGTCTTGAACCCACGGACCA |
| SEQ ID NO:4 | ITS SEQ of *Kluyveromyces lactis* AMCC 31347 | TCCGGGGAATCTACCTGATTTGAGGTCAACTTTG AGAGTTTTGATTAAGCCGTATGCCTCAAGGAGA CAAACACCAGCGAGTCTTTATAACACCTATAAG CTCATTGACCCTAGCTTACCACGAATTGGCGCAA ACCTAAGACGTAGATGTGCAAGAGTCGAGTCCA TAGACTTGACACGCAGCCCTGCTCACGCAGAAG |

24

(continued)

| SEQ ID NO. | SEQ Descriptions | SEQ |
|---|---|---|
| | | GCAACGGCTAGCCACTTTCAAGTTAACCCGAAA AACGAGTATCACTCACTACCAAACCCAAAGGTT TGAGAGAGAAATGACGCTCAAACAGGCATGCCC CCTGGAATACCAGAGGGCGCAATGTGCGTTCAA AGATTTGATGATTCACGAAAATCTGCAATTCACA ATACATATCGCAATTCGCTGCGTTCTTCATCGAT GCGAGAACCAAGAGATCCGTTGTTGAAAGTTTT GAATATTAAATTTCTTGATAAATAGTTTTTCATA ATGCAAAATGTTGTTTGTGTTTATGTCCACTGGA GAGACGAGCTCTCCAGGGAAGTAGTTCATAGAG AAAAAACTCCATTGTGTTTAGGATGAGAGAAAG AAAACTGATAGCAGAAAATCAAGAATTAGCCGC GCAATTAAGCGCAGGCCATATTCAGCGATTCCC CCAGTAATCTACTCATTCATAATCTTTAATGATC CTTCCGCAGGTTCACCTACGGAAACCTTGTTACG ATTTTTTACCTCCAA |

**Claims**

1. A method for preparing a yeast product using a high-lactic acid industrial by-product, **characterized in that** comprising the following steps:

   (1) inoculating an activated yeast strain into a primary seed culture medium for fermentation culture to obtain a primary seed liquid; wherein the yeast strain is selected from one or two or more of the group consisting of *Kluyveromyces marxianus, Kluyveromyces lactis, Pichia kudriavzeii,* and *Candida tropicalis;*
   (2) inoculating the primary seed liquid into a secondary seed culture medium for fermentation culture to obtain a secondary seed liquid;
   (3) inoculating the secondary seed liquid into a fermentation culture medium for fermentation culture, wherein the fermentation culture medium comprises the high-lactic acid industrial by-product and/or a modified product of the high-lactic acid industrial by-product; and
   (4) separating the fermentation broth obtained in step (3) to obtain a yeast product, or drying the fermentation broth obtained in step (3) to obtain a yeast product.

2. The method according to claim 1, **characterized in that** the high-lactic acid industrial by-product of step (3) comprises one or two or more of the group consisting of corn steep liquor, Distillers Dried Grains with Solubles (DDGS), Baijiu yellow water or pickling water, preferably corn steep liquor.

3. The method according to claim 1 or 2, **characterized in that** the fermentation culture medium further comprises a nutrient, wherein the nutrient comprises a trace element and a vitamin.

4. The method according to claim 3, **characterized in that** the trace element is selected from one or two or more of the group consisting of copper, calcium, iron, zinc, magnesium, and potassium, preferably copper and/or calcium; preferably, the vitamin is selected from one or two or more of the group consisting of vitamin B1, vitamin B2, vitamin B3, vitamin B6, pantothenic acid, and vitamin B7, preferably one or two or more of the group consisting of vitamin B1, pantothenic acid, and vitamin B7.

5. The method according to claim 3 or 4, **characterized in that** the nutrient comprises one or two or more of the group consisting of copper 2-5 ppm, calcium 400-1000 ppm, vitamin B1 10-30 ppm, pantothenic acid 15-30 ppm, and vitamin B7 0.5-1.5 ppm, based on the total weight of the fermentation culture medium.

6. The method according to any one of claims 1-5, **characterized in that** the modified product of the high-lactic acid industrial by-product is prepared by a process comprising the following steps: subjecting the high-lactic acid industrial by-product to enzymolysis with cellulase, enzymolysis with protease, and enzyme inactivation.

7. The method according to claim 6, **characterized in that** the cellulase is added in an amount of 3-6 wt%o, preferably 4-6 wt%o, based on the dry weight of the high-lactic acid industrial by-product; preferably, the cellulase enzymolysis temperature is 55-65°C; preferably the enzymolysis time is 3-6 hours.

8. The method according to claim 6 or 7, **characterized in that** the protease is added in an amount of 2-4 wt%o, preferably 2-3 wt%o, based on the dry weight of the high-lactic acid industrial by-product; preferably, the protease enzymolysis temperature is 55-65°C; preferably the enzymolysis time is 3-6 hours.

9. The method according to any one of claims 6-8, **characterized in that** the enzyme inactivation comprises heating to 70-90°C and holding for 20-40 min.

10. The method according to any one of claims 1-9, **characterized in that** the fermentation culture of step (3) uses a stirred fermenter, with a stirring speed of 100-600 rpm, and preferably an aeration ratio of 1: 1-2; alternatively, the fermentation culture of step (3) uses an air-lift fermenter with an aeration ratio of 1: 10-70; preferably, the fermentation culture temperature of step (3) is 25-35°C, and preferably, the culture time is 12-48 hours.

11. The method according to any one of claims 1-10, **characterized in that** the separation in step (4) comprises one or two or more of the group consisting of centrifugation, suction filtration, pressure filtration or plate-and-frame filtration, preferably centrifugation, more preferably the centrifugation speed is 3000-10000 rpm, even more preferably the centrifugation time is 5-20 min.

12. The method according to any one of claims 1-11, **characterized in that** comprising the step of washing and/or drying the yeast product separated in step (4).

13. The method according to any one of claims 1-12, **characterized in that** the primary seed culture medium of step (1) is a YPD liquid medium; preferably, the fermentation culture temperature of step (1) is 25-35°C, preferably the fermentation culture time is 12-30 hours; more preferably, the speed is 150-220 rpm.

14. The method according to any one of claims 1-13, **characterized in that** the secondary seed culture medium of step (2) comprises glucose 5-8%, yeast leachate 0.3-0.6%, ammonium sulfate 1-2%, zinc sulfate 0.2-0.4%, potassium dihydrogen phosphate 0.4-0.6%, and magnesium sulfate 0.4-0.6% by mass volume percentage; preferably, the fermentation culture temperature of step (2) is 25-35°C; preferably the culture time is 12-36 hours; more preferably, the fermentation culture pH is 4.5-5.5; preferably the aeration is 2-4 L/min.

15. A yeast product obtainable by the method according to any one of claims 1-14, **characterized in that** the yeast product comprises 30-68% of protein by weight, preferably 50-68% of protein by weight; preferably, the yeast product comprises 1.5-9.0% of trehalose; more preferably, the yeast product comprises 2.0-4.0% of phosphorus pentoxide.

16. The yeast product according to claim 15, **characterized in that** the yeast product is in the form of a powder, paste, or liquid.

17. Use of the yeast product according to claim 15 or 16 in feed protein, food seasoning or fermentation of industrial nitrogen sources.

Fig. 1

Fig. 2

Fig. 3

Fig. 4

## INTERNATIONAL SEARCH REPORT

| International application No. |
|---|
| **PCT/CN2023/140941** |

### A. CLASSIFICATION OF SUBJECT MATTER

C12N 1/16(2006.01)i; A23K 10/12(2016.01)i; A23K 10/18(2016.01)i; A23K 10/30(2016.01)i; A23K 10/37(2016.01)i; A23L 27/24(2016.01)i

According to International Patent Classification (IPC) or to both national classification and IPC

### B. FIELDS SEARCHED

Minimum documentation searched (classification system followed by classification symbols)

IPC:C12N,A23K,A23L

Documentation searched other than minimum documentation to the extent that such documents are included in the fields searched

Electronic data base consulted during the international search (name of data base and, where practicable, search terms used)

DWPI, WPABS, CNTXT, USTXT, JPTXT, EPTXT, WOTXT, VEN, CNKI, 万方数据资源系统, WANFANG DATA: 申请人, 发明人, 高乳酸工业副产物, 酵母, 发酵, 玉米浆, 酒糟, 白酒黄水, 泡菜水, 微量元素, lactate, yeast, ferment, corn steep liquor, distillers, microelement

### C. DOCUMENTS CONSIDERED TO BE RELEVANT

| Category* | Citation of document, with indication, where appropriate, of the relevant passages | Relevant to claim No. |
|---|---|---|
| Y | CN 103642857 A (FUJIAN BEICHEN BIOTECHNOLOGY CO., LTD.) 19 March 2014 (2014-03-19) <br> description, embodiment 1 | 1-17 |
| Y | CN 115418375 A (WUHAN SUNHY BIOLOGICAL CO., LTD.) 02 December 2022 (2022-12-02) <br> description, paragraphs 5 and 6 | 1-17 |
| Y | CN 109536541 A (JILIN COFCO BIO-CHEMICAL CO., LTD. et al.) 29 March 2019 (2019-03-29) <br> claims 1-4 | 1-17 |
| Y | CN 111593078 A (COFCO NUTRITION AND HEALTH RESEARCH INSTITUTE CO., LTD. et al.) 28 August 2020 (2020-08-28) <br> claims 1-4 | 1-17 |
| A | EP 0017853 A2 (PHILLIPS PETROLEUM COMPANY) 29 October 1980 (1980-10-29) <br> claims | 1-17 |

☐ Further documents are listed in the continuation of Box C.　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 March 2024** | **01 April 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/140941**

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

   ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

**INTERNATIONAL SEARCH REPORT**
Information on patent family members

International application No.

**PCT/CN2023/140941**

| Patent document cited in search report | | | Publication date (day/month/year) | Patent family member(s) | | | Publication date (day/month/year) |
|---|---|---|---|---|---|---|---|
| CN | 103642857 | A | 19 March 2014 | None | | | |
| CN | 115418375 | A | 02 December 2022 | None | | | |
| CN | 109536541 | A | 29 March 2019 | None | | | |
| CN | 111593078 | A | 28 August 2020 | None | | | |
| EP | 0017853 | A2 | 29 October 1980 | MY | 8500218 | A | 31 December 1985 |
| | | | | GB | 2047268 | A | 26 November 1980 |
| | | | | GB | 2047268 | B | 15 June 1983 |
| | | | | DE | 3069485 | D1 | 29 November 1984 |
| | | | | MX | 167363 | B | 18 March 1993 |
| | | | | MX | 7349 | E | 19 July 1988 |
| | | | | KR | 830002869 | A | 31 May 1983 |
| | | | | KR | 860000893 | B1 | 16 July 1986 |
| | | | | DK | 157480 | A | 13 October 1980 |
| | | | | ES | 8105384 | A1 | 16 April 1981 |
| | | | | NO | 801065 | L | 13 October 1980 |
| | | | | NO | 168370 | B | 04 November 1991 |
| | | | | NO | 168370 | C | 12 February 1992 |
| | | | | GB | 8300170 | D0 | 09 February 1983 |
| | | | | GB | 2124652 | A | 22 February 1984 |
| | | | | GB | 2124652 | B | 01 August 1984 |
| | | | | EP | 0017853 | A3 | 28 January 1981 |
| | | | | EP | 0017853 | B1 | 24 October 1984 |
| | | | | EP | 0017853 | B2 | 04 April 1990 |
| | | | | SG | 79583 | G | 03 August 1984 |
| | | | | CA | 1149298 | A | 05 July 1983 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310540113X
- CN 113455584 A
- CN 201710532216
- CN 109207384 A
- CN 202111509846
- CN 114107077 A
- GB T235302009 A